# EUROPEAN PATENT APPLICATION

(11) **EP 2 186 890 A1**
(43) Date of publication of application: **19.05.2010**
(21) Application number: 08791827.2
(22) Date of filing: 29.07.2008
(51) Int. Cl.: C12N 15/00, C12N 1/16, C12N 1/20, C12N 15/09, C12P 7/06, C12N 11/02

(54) **METHOD FOR PRODUCTION OF BIOFILM**

(30) Priority: 31.07.2007 JP 2007198792; 28.02.2008 JP 2008047195
(71) Applicant: Nihon University, Tokyo 102-8275 (JP)
(72) Inventor: MORINAGA, Yasushi, Tokyo 102-8275 (JP); FURUKAWA, Soichi, Tokyo 102-8275 (JP); OGIHARA, Hirokazu, Tokyo 102-8275 (JP); YAMASAKI, Makari, Tokyo 102-8275 (JP)
(74) Representative: Ford, Hazel
(86) International application number: PCT/JP2008/063592
(87) International publication number: WO 2009/017124

(57) **Abstract**

For the purpose of forming a biofilm having amount and strength sufficient for the immobilization of a cell mass, the present invention provides a method for producing a biofilm, **characterized in that** it comprises co-culturing lactic acid bacteria belonging to Lactobacillus plantarum with yeast.

## Description

### Technical Field

The present invention relates to a method for producing a biofilm by the co-culture of lactic acid bacteria with yeast, a biofilm produced by the method, and a method for producing a useful substance by the co-culture of lactic acid bacteria with yeast.

### Background Art

A method for producing a useful substance using yeast, in which an immobilized cell mass is used, has been known. As a method for preparing an immobilized cell mass of yeast, an immobilization method using a gel support such as alginic acid and other methods have been known. On the other hand, the presence of a microorganism biofilm has been known. In many cases, however, only the problem of such biofilm regarding food sanitation has been reported. There have been almost no examples in which such biofilm intends to be used in production of useful substances.

Recently, the present inventors have reported that a substance contained in a culture filtrate of lactic acid bacteria belonging to Lactobacillus casei (Lactobacillus casei var. rhamnosus IFO 3831) promotes the formation of a biofilm with yeast belonging to Saccharomyces cerevisiae (Saccharomyces cerevisiae Kyokai No. 10) (Non-Patent Document 1).
Non-Patent Document 1: Takehito Kawarai et al., Summary of Annual Meeting of JSBBA 2007, the Japan Society for Bioscience, Biotechnology, and Agrochemistry, p. 169

### Disclosure of the Invention

### Problems to be Solved by the Invention

Preparation of an immobilized cell mass of yeast has required an expensive support such as alginic acid, and it has also required extremely complicated steps, such as the immobilization of a cell mass on a support and the filling of a reactor with the cell mass-immobilized support. On the other hand, a biofilm is also formed by singly culturing yeast. However, since the thus formed biofilm is not sufficient in terms of amount and strength, it has not been used in the immobilization of a cell mass. Moreover, even if the biofilm formation-promoting method described in Non-Patent Document 1 is applied, the amount of a biofilm formed is not sufficient, and thus the formed biofilm has not been used, either, in the immobilization of a cell mass.

The present invention has been made under the aforementioned technical background. It is an object of the present invention to provide a means for forming a biofilm having amount and strength sufficient for the immobilization of a cell mass.

### Means for Solving the Problems

As a result of intensive studies directed towards achieving the aforementioned object, the present inventors have found that the amount of a biofilm formed is significantly increased by culturing lactic acid bacteria belonging to Lactobacillus plantarum and yeast such that they are directly contacted with each other.

Non-Patent Document 1 has already disclosed that the amount of a biofilm formed is increased by co-culturing lactic acid bacteria with yeast. However, the lactic acid bacteria used in Non-Patent Document 1 is Lactobacillus casei, and thus the invention described in Non-Patent Document 1 greatly differs from the present invention, in which Lactobacillus plantarum is used. Moreover, the effect of promoting the formation of a biofilm is also greatly different between the present invention and the invention described in Non-Patent Document 1. The aforementioned effect of the present invention is much higher than that of the invention described in Non-Patent Document 1. Furthermore, in the invention described in Non-Patent Document 1, cell masses are not directly contacted with each other, and the amount of a biofilm formed can be increased also using a culture filtrate. In contrast, in the present invention, the amount of a biofilm formed cannot be increased, unless cell masses are directly contacted with each other. In this respect, the two inventions differ from each other.

Still further, the inventors have also found that, in the formed biofilm, lactic acid bacterial cells adhere to the surface of a solid, but yeast cells do not adhere to such solid surface but are immobilized on the solid surface via lactic acid bacteria.

The present invention has been completed based on the aforementioned findings.

Specifically, the present invention provides the following (1) to (19).
(1) A method for producing a biofilm, **characterized in that** it comprises co-culturing lactic acid bacteria belonging to Lactobacillus plantarum with yeast.
(2) The method for producing a biofilm according to (1) above, **characterized in that** the lactic acid bacteria belonging to Lactobacillus plantarum are ML11-11 deposited under accession No. NITE BP-376.
(3) The method for producing a biofilm according to (1) above, **characterized in that** the lactic acid bacteria belonging to Lactobacillus plantarum have 16S rDNA consisting of a nucleotide sequence showing homology of 97% or more with the nucleotide sequence shown in SEQ ID NO:1.
(4) The method for producing a biofilm according to any one of (1) to (3) above, **characterized in that** the yeast belongs to Saccharomyces cerevisiae.
(5) The method for producing a biofilm according to any one of (1) to (3) above, **characterized in that** the yeast has 18S rDNA consisting of a nucleotide sequence showing homology of 97% or more with the nucleotide sequence shown in SEQ ID NO:2.
(6) A biofilm produced by the method according to any one of (1) to (5) above.
(7) A method for producing a useful substance, using lactic acid bacteria that belong to Lactobacillus plantarum and produce such useful substance, or yeast that produces such useful substance, **characterized in that** it comprises co-culturing the lactic acid bacteria with the yeast and collecting a useful substance from the culture.
(8) The method for producing a useful substance according to (7) above, **characterized in that** the lactic acid bacteria and the yeast are converted to an immobilized cell mass, using a biofilm formed by the co-culture of both types of microorganisms.
(9) The method for producing a useful substance according to (7) or (8) above, **characterized in that** the useful substance is ethanol.
(10) The method for producing a useful substance according to any one of (7) to (9) above, **characterized in that** the lactic acid bacteria belonging to Lactobacillus plantarum are ML11-11 deposited under accession No. NITE BP-376.
(11) The method for producing a useful substance according to any one of (7) to (9) above, **characterized in that** the lactic acid bacteria belonging to Lactobacillus plantarum have 16S rDNA consisting of a nucleotide sequence showing homology of 97% or more with the nucleotide sequence shown in SEQ ID NO:1.
(12) The method for producing a useful substance according to any one of (7) to (11) above, **characterized in that** the yeast belongs to Saccharomyces cerevisiae.
(13) The method for producing a useful substance according to any one of (7) to (11) above, **characterized in that** the yeast has 18S rDNA consisting of a nucleotide sequence showing homology of 97% or more with the nucleotide sequence shown in SEQ ID NO:2.
(14) A biofilm comprising yeast and lactic acid bacteria, **characterized in that** the lactic acid bacterial cells adhere to a solid surface and yeast cells are immobilized on the solid surface via the lactic acid bacterial cells.
(15) The biofilm according to (14) above, **characterized in that** the lactic acid bacteria are ML11-11 deposited under accession No. NITE BP-376.
(16) The biofilm according to (14) above, **characterized in that** the lactic acid bacteria have 16S rDNA consisting of a nucleotide sequence showing homology of 97% or more with the nucleotide sequence shown in SEQ ID NO:1.
(17) The biofilm according to any one of (14) to (16) above, **characterized in that** the yeast belongs to Saccharomyces cerevisiae.
(18) The biofilm according to any one of (14) to (16) above, **characterized in that** the yeast has 18S rDNA consisting of a nucleotide sequence showing homology of 97% or more with the nucleotide sequence shown in SEQ ID NO:2.
(19) Lactobacillus plantarum ML11-11 (Accession No. NITE BP-376).

### Advantages of the Invention

According to the present invention, it becomes possible to efficiently produce a strong biofilm. In addition, utilizing the thus produced biofilm, it becomes possible to produce useful substances such as ethanol, using an immobilized cell mass, without using a support such as alginic acid.

### Brief Description of the Drawings

Figure 1 is a view showing the amount of a biofilm formed, when lactic acid bacteria and yeast isolated from pot vinegar are co-cultured;
Figure 2 is a view showing the amount of a biofilm formed, when Y11-43 and ML11-11 are cultured under various conditions;
Figure 3 is a view showing the amount of a biofilm formed, when Y11-43 and lactic acid bacteria other than ML11-11 are co-cultured;
Figure 4 is a view showing the amount of a biofilm formed, when ML11-11 and yeast other than Y11-43 are co-cultured;
Figure 5 is a view showing the amount of a biofilm formed, when S. cerevisiae Kyokai No. 10 and L. casei subsp. rhamnosus IFO 3831 are co-cultured;
Figure 6 is a view showing the amount of a biofilm formed, when ML11-11 and diploid yeast or monoploid yeast are co-cultured;
Figure 7 is a view showing the amount of carbon dioxide generated by a co-culture biofilm in various sugar concentrations;
Figure 8 is a view showing the amount of carbon dioxide generated by singly cultured yeast cells in various sugar concentrations;
Figure 9 is a view showing a change in the number of yeast cells due to washing;
Figure 10 is a view showing the fine structure of a biofilm formed by co-culture (1). Yeast cells adhere to the surface of a solid in the form of a layer;
Figure 11 is a view showing the fine structure of a biofilm formed by co-culture (2). Lactic acid bacterial cells (small size) singly adhere to the surface of a solid, but yeast cells (large size) do not singly adhere to the solid surface;
Figure 12 is a view showing the fine structure of a biofilm formed by co-culture (3). Lactic acid bacterial cells (small size) are intertwined with yeast cells (large size);
Figure 13 is a view showing the fine structure of singly cultured yeast. The yeast cells hardly adhere to the surface of a solid by themselves, and they are easily removed by washing;
Figure 14 is a view showing the fine structure of singly cultured lactic acid bacteria. The lactic acid bacteria adhere to the surface of a solid by themselves to a certain extent;
Figure 15 is a view showing the state of a glass surface 4 hours after the initiation of the co-culture of lactic acid bacteria with yeast (initial stage of adhesion). Only the lactic acid bacterial cells adhere to the glass surface, and yeast cells hardly adhere thereto;
Figure 16 is a view showing the state of a glass surface 8 hours after the initiation of the co-culture of lactic acid bacteria with yeast (growth stage to become a biofilm). The lactic acid bacterial cells form a film-like aggregate. The number of the yeast cells is small, and the yeast cells adhere to the upper portion of an aggregate of lactic acid bacteria, but do not adhere to the glass surface;
Figure 17 is a view showing the state of a glass surface 12 hours after the initiation of the co-culture of lactic acid bacteria with yeast (a grown biofilm). A large number of yeast cells begin to adhere onto a biofilm, but there are no yeast cells that directly adhere to a glass surface;
Figure 18 is a view showing the state of a glass surface 16 hours after the initiation of the co-culture of lactic acid bacteria with yeast (a completed biofilm). The yeast cells are intertwined with the lactic acid bacterial cells, and the yeast cells are closely together with the lactic acid bacterial cells, so as to form a high-cell-density biofilm; and
Figure 19 is a view schematically showing a biofilm formation mechanism involving the co-culture of lactic acid bacteria with yeast.

### Best Mode for Carrying Out the Invention

The present invention will be described in detail below.

The method for producing a biofilm of the present invention is **characterized in that** lactic acid bacteria that belong to Lactobacillus plantarum are co-cultured with yeast.

As such lactic acid bacteria, ML11-11 is preferably used. This strain has been isolated by the present inventors. The strain was deposited with the NITE Patent Microorganisms Depositary (NPMD) (2-5-8, Kazusa Kamatari, Kisarazu-shi, Chiba-ken, Japan) under accession No. NITE BP-376 (deposition date: July 10, 2007). Strains having 16S rDNA showing high homology with the 16S rDNA of ML11-11 (the nucleotide sequence is as shown in SEQ ID NO:1) are related to ML11-11. It is highly likely that such related strains promote the formation of a biofilm by the co-culture of the strains with yeast, as in the case of ML11-11. Accordingly, such related strains may also be used instead of ML11-11. The term "high homology" is used herein to mean generally homology of 97% or more, preferably homology of 99% or more, and most preferably homology of 100%. In addition, even if a certain strain is a strain other than ML11-11 or other than strains related to such ML11-11, it can be used in the present invention, as far as it is a strain that belongs to Lactobacillus plantarum and is capable of promoting the formation of a biofilm as a result of the co-culture of it with yeast.

As yeast, Y11-43 isolated by the present inventor is preferably used. Strains having 18S rDNA showing high homology with the 18S rDNA of Y11-43 (the nucleotide sequence is as shown in SEQ ID NO:2) are related to Y11-43. It is highly likely that such related strains exhibit action to promote the formation of a biofilm as a result of the co-culture of it with lactic acid bacteria, as in the case of Y11-43. Accordingly, such related strains may also be used instead of Y11-43. The term "high homology" is used herein to mean generally homology of 97% or more, preferably homology of 99% or more, and most preferably homology of 100%. In addition to Y11-43 and the related strains thereof, known yeasts belonging to Saccharomyces cerevisiae, such as Saccharomyces cerevisiae X2180-1A, Saccharomyces cerevisiae Kyokai No. 7, and Saccharomyces cerevisiae Kyokai No. 10, can also be used. Moreover, yeasts belonging to genus Saccharomyces other than Saccharomyces cerevisiae and yeasts belonging to genera other than genus Saccharomyces may also be used, as far as they have the effect of promoting the formation of a biofilm as a result of the co-culture of them with lactic acid bacteria. Examples of such yeast belonging to genus Saccharomyces other than Saccharomyces cerevisiae include Saccharomyces pasteurianus and Saccharomyces bayanus. Examples of such yeast belonging to genera other than genus Saccharomyces include yeasts belonging to genus Schizosaccharomyces and genus Candida.

The method of co-culturing lactic acid bacteria with yeast is not particularly limited, as long as it enables the proliferation of both types of microorganisms. As a medium, a medium containing yeast extract, peptone, glucose and the like, such as a YPD medium (1% yeast extract, 2%, peptone, and 2% glucose), can be used. The culture temperature is not particularly limited. It is preferably 25°C to 35°C, and more preferably 28°C to 30°C.

The biofilm of the present invention comprises yeast and lactic acid bacteria. The present biofilm is **characterized in that** lactic acid bacterial cells adhere to the surface of a solid and in that yeast cells are immobilized on the solid surface via the lactic acid bacterial cells. The biofilm of the present invention can be produced by co-culturing lactic acid bacteria belonging to Lactobacillus plantarum with yeast according to the aforementioned method for producing a biofilm. The present biofilm may also be produced by co-culturing lactic acid bacteria other than those belonging to Lactobacillus plantarum with yeast.

The method for producing a useful substance of the present invention is formed by applying the aforementioned method for producing a biofilm. The present method is **characterized in that** it comprises co-culturing lactic acid bacteria belonging to Lactobacillus plantarum with yeast and collecting a useful substance from the culture.

The types of lactic acid bacteria and yeast used and the co-culture method may be the same as those applied to the method for producing a biofilm.

In this method for producing a useful substance, since a biofilm is formed by the co-culture of lactic acid bacteria with yeast, microorganisms can be converted to an immobilized cell mass without using a gel support or the like.

Either lactic acid bacteria or yeast may produce such useful substance. Examples of a useful substance include: lactic acid, bacteriocin, various types of peptides and various types of enzymes, which are produced by lactic acid bacteria; and ethanol and various types of enzymes produced by yeast. Among such useful substances, ethanol is most preferable for the following reason. In ethanol fermentation, if the concentration of ethanol becomes high in fermented mash, the activity of yeast decreases, and thereby, the fermentative reaction does not progress. Thus, the ethanol concentration in the fermented mash cannot be set at high. By the method of the present invention, however, since yeast is protected by a biofilm, it is considered to be resistant to ethanol. Accordingly, fermentation progresses even in an ethanol concentration higher than the conventional concentration, so that it is considered that fermented mash having a higher ethanol concentration can be obtained. Moreover, in ethanol fermentation, a continuous fermentation method is often used to enhance productivity. In order to maintain a cell concentration at a stable level in the system, the immobilization of a yeast cell mass and a step of recovering a yeast cell mass leaked to the outside of the system by centrifugation or the like and then returning it again into the system are carried out. The formation of a biofilm based on the method of the present invention is greatly advantageous in that it does not only become easy to immobilize a yeast cell mass as a biofilm, but also the sedimentation property of the yeast cell mass is improved, and as a result, it becomes easy to recover a yeast cell mass leaked to the outside of the system by centrifugation or the like.

### Examples

The present invention will be described more in detail in the following examples.

### [Example 1] Screening for biofilm-forming strain

Lactic acid bacteria and yeast isolated from a fermented sample of Fukuyama pot vinegar from Fukuyama region, Kagoshima prefecture, were cultured together, and the amount of a biofilm formed was then examined.

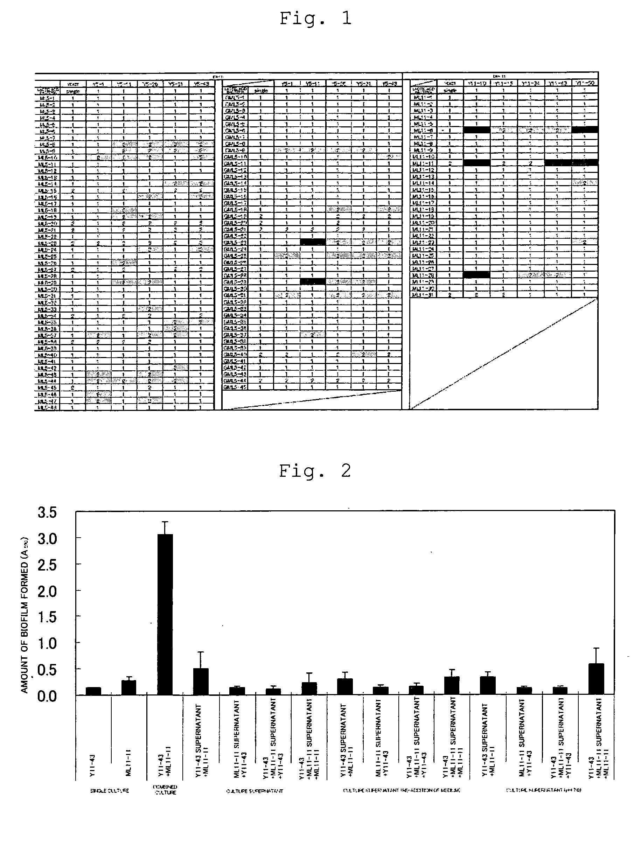

In the autumn 2006, such pot vinegar was prepared, and on Day 5 and Day 11, samples were collected (wherein the samples were furnished from Mr. Hidefumi Date of Date Jozo G.K.). Lactic acid bacteria and yeast isolated from the sample were co-cultured. The culture was carried out at 30°C. Twenty-four hours after the initiation of the culture, a biofilm was stained with crystal violet, and the amount of the biofilm formed was evaluated at 5 stages (1 to 5 stages). The results are shown in Figure 1.

The amount of a biofilm formed when several types of combinations of lactic acid bacteria with yeast were subjected to co-culture was larger than that when either lactic acid bacteria or yeast was singly cultured. When lactic acid bacteria ML11-11 was co-cultured with yeast Y11-43, the largest amount of a biofilm was formed.

### [Example 2] Identification of ML11-11 and Y11-43

When strains were identified using 16S ribosome DNA and 18S ribosome DNA. As a result, lactic acid bacteria ML11-11 was identified to be Lactobacillus plantarum, and yeast Y11-43 was identified to be Saccharomyces cerevisiae. The nucleotide sequence of 16S ribosome DNA of lactic acid bacteria ML11-11 is shown in SEQ ID NO:1, and the nucleotide sequence of 18S ribosome DNA of yeast Y11-43 is shown in SEQ ID NO:2.

### [Example 3] Analysis of conditions of ML11-11 and Y11-43 for formation of biofilm

Yeast Y11-43 and lactic acid bacterial ML11-11 were pre-cultured at 27°C for 24 hours, respectively, in a YPD medium and an MRS medium (10.0g of Proteose peptone No. 3; 10.0 g of Beef Extract; 5.0 g of Yeast Extract; 20.0 g of Dextrose, 1.0 g of Polysorbate 80; 2.0 g of Ammonium citrate; 5.0 g of Sodium Acetate; 0.1 g of Magnesium Sulfate; 0.05 g of Manganese Sulfate; and 2.0 g of Dipotassium Phosphate (in 1 L)). The pre-culture solutions of Y11-43 and ML11-11 were each inoculated in a YPD medium, and the obtained mixture was dispensed to a 96-well titer plate in an amount of 100 µL per well. In a case in which only one type of strain was to be inoculated, the pre-culture solution was inoculated to the YPD medium in an amount of 1/100 of the medium per strain. In a case in which two types of strains were to be inoculated, the pre-culture solution was inoculated to the YPD medium in an amount of 1/200 of the medium per strain. After completion of the inoculation of the pre-cultured solution, static culture was carried out at 30°C for 24 hours, and a biofilm was then stained by a crystal violet (0.1%) staining method. A pigment was eluted from the stained biofilm. The absorbance of the eluted pigment was measured, and it was evaluated as the amount of a biofilm formed.

The results obtained by analyzing the conditions of yeast Y11-43 and lactic acid bacteria ML11-11 for the formation of a biofilm are shown in Figure 2. The formation of a biofilm was significantly promoted when both types of microorganisms were co-cultured in a single medium. On the other hand, when a supernatant obtained by sterilizing a single culture solution of ML11-11 with a filter was added to a single culture medium of Y11-43, the formation of a biofilm was not promoted. It was likely that the formation of a biofilm was suppressed due to lack of a nutrient or a decrease in pH under such supernatant-added conditions. Thus, this respect was analyzed. However, although medium ingredients as well as an ML11-11 supernatant were added to a medium to be used in the single culture of Y11-43, or the pH was readjusted to be pH 7.0, biofilm formation was not promoted.

The aforementioned results demonstrate that the direct contact of yeast cells with lactic acid bacterial cells is required for biofilm formation.

### [Example 4] Evaluation of amount of biofilm formed by strains other than pot vinegar-isolated strains

### (Used strains)

The following strains were used as strains other than Y11-43 and ML11-11.
Lactic acid bacteria: Leuconostoc mesenteroides subsp.
mesenteroides IAM 1046
   Lactobacillus sakei IFO 3541
   Lactobacillus casei subsp.
rhamnosus IFO 3831
Yeast: Saccharomyces cerevisiae X2180-1A
   Saccharomyces cerevisiae Kyokai No. 7
   Saccharomyces cerevisiae Kyokai No. 10

### (Media and culture conditions)

A YPD medium was used for the pre-culture of yeast, and an MRS medium was used for the pre-culture of lactic acid bacteria. The pre-culture was carried out at 27°C for 24 hours.

### (Evaluation of amount of biofilm formed)

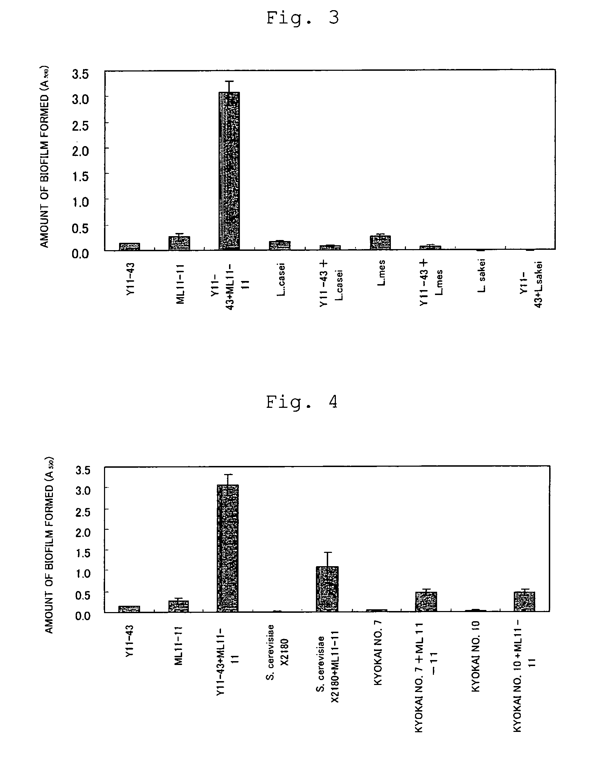

The pre-culture solutions of yeast and lactic acid bacteria were each inoculated to a YPD medium, and the obtained mixture was dispensed to a 96-well titer plate in an amount of 100 µL per well. In a case in which only one type of strain was to be inoculated, the pre-culture solution was inoculated to the YPD medium in an amount of 1/100 of the medium per strain. In a case in which two types of strains were to be inoculated, the pre-culture solution was inoculated to the YPD medium in an amount of 1/200 of the medium per strain. After completion of the inoculation of the pre-cultured solution, static culture was carried out at 30°C for 24 hours, and a biofilm was then stained by a crystal violet (0.1%) staining method. A pigment was eluted from the stained biofilm. The absorbance of the eluted pigment was measured, and it was evaluated as the amount of a biofilm formed. The amounts of biofilms formed when Y11-43 was co-cultured with lactic acid bacteria other than ML11-11 are shown in Figure 3, and the amounts of biofilms formed when ML11-11 was co-cultured with yeast other than Y11-43 are shown in Figure 4.

When lactic acid bacteria other than ML11-11 were co-cultured with Y11-43, the amount of a biofilm formed was not increased. On the other hand, when ML11-11 was co-cultured with yeasts other than Y11-43, a significant increase in the amount of a biofilm formed was confirmed. In particular, when S. cerevisiae X2180 was used, the manner of forming a biofilm was extremely similar to the case of using Y11-43, although the obtained numerical value was lower than that in the case of using Y11-43. From these results, it was assumed that an increase in the amount of a biofilm formed by Y11-43 and ML11-11 was highly likely caused by the action of ML11-11. Moreover, it is considered that such ML11-11 generally promotes the formation of a biofilm by budding yeast belonging to S. cerevisiae.

### [Comparative Example]

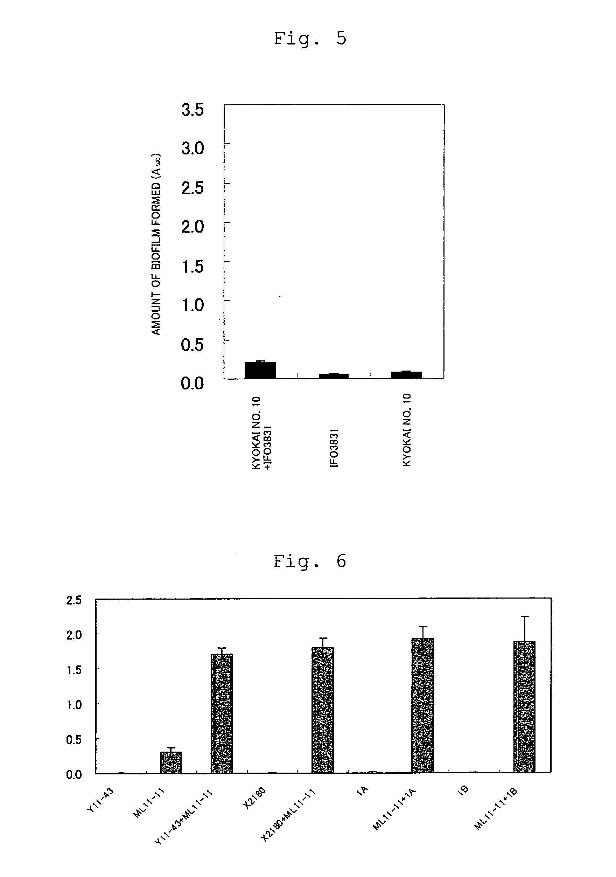

Saccharomyces cerevisiae Kyokai No. 10 and Lactobacillus casei subsp. rhamnosus IFO 3831, which are described in Non-Patent Document 1 (Takehito Kawarai et al., Summary of Annual Meeting of JSBBA 2007, the Japan Society for Bioscience, Biotechnology, and Agrochemistry, p. 169), were subjected to co-culture under the same conditions as those in Example 4. Thereafter, the amount of a biofilm formed was evaluated. The results are shown in Figure 5.

As shown in Figure 5, the amount of a biofilm formed by the co-culture of Saccharomyces cerevisiae Kyokai No. 10 with Lactobacillus casei subsp. rhamnosus IFO 3831 was 1/10 of the amount of a biofilm formed by the co-culture of Y11-43 with ML11-11, or lower than 1/10 of the same above amount.

### [Example 5] Formation of biofilm by haploid yeast

A majority of actually used yeasts are polyploidy yeasts. Thus, whether or not the method of the present invention can be applied in the case of using different polyploidy yeasts was studied by the same method as that applied in Example 4. As models in the experiment, there were used diploid laboratory yeast X2180, haplotype 1A as a haploid thereof, and different haplotype 1B. These yeasts were each compared with wild-type yeast Y11-43. As a result, it could be confirmed that both haploid and diploid yeasts form a biofilm as a result of the co-culture of them with lactic acid bacteria ML11-11 in the completely same manner as that of the wild-type yeast.
It was clarified that biofilm formation occurs, regardless of the ploidy of yeast (Figure 6).

### [Example 6] Influence of sugar concentration on generation of carbon dioxide

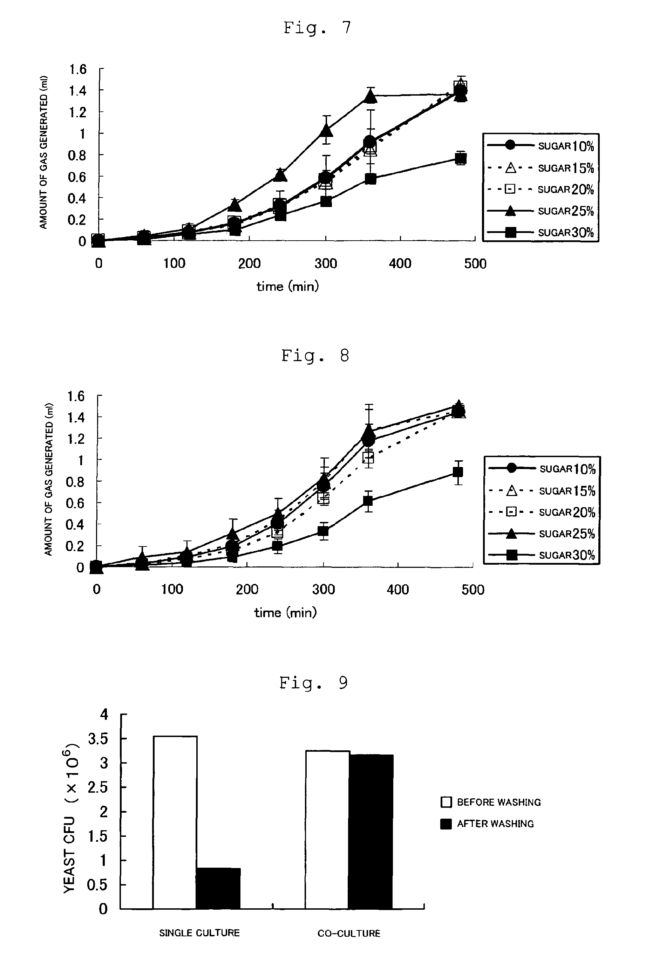

Using biofilm-forming cells generated as a result of co-culture and singly cultured yeast cells (floating cells), the amounts of carbon dioxide generated in media having glucose concentrations of 10% to 30% were examined. Y11-43 yeast cells were inoculated to a YPD medium, singly or together with ML11-11 lactic acid bacterial cells, followed by static culture at 30°C for 24 hours. Thereafter, floating cells (single culture) and biofilm-forming cells (co-culture) were recovered by centrifugation. After washing, the recovered cells were added to YP media containing 10% to 30% glucoses. The obtained mixtures were each subjected to static culture at 30°C, and the amount of gas generated was then measured. As a result, it was found that the amounts of carbon dioxide generated in media having glucose concentration of containing 10% and 20% were almost equivalent in both cases of using a biofilm and singly cultured yeast cells, and that the generation of carbon dioxide tended to be suppressed in a medium having glucose concentrations of 30% (Figures 7 and 8). From these results, it was confirmed that biofilm-forming cells generate carbon dioxide from sugar, as with floating cells obtained by a single culture of yeast. These results demonstrate that the use of a biofilm enables the same generation of ethanol as in the case of a single culture.

### [Example 7] Stability of yeast cells in co-cultured biofilm

In order to use a biofilm as immobilized cells, the stability of the biofilm against mechanical shock becomes important. Hence, the stability of a biofilm formed by the co-culture of yeast Y11-43 with lactic acid bacteria ML11-11 against washing was evaluated.

Yeast Y11-43 was co-cultured with lactic acid bacteria ML11-11 in 20 ml of a YPD medium placed in a Petri dish under static conditions, so that a biofilm was formed on a stainless steel board (each side: 25 mm). After completion of the culture, the stainless steel board, on which a biofilm had been formed, was immersed twice in 100 ml of a sterilized normal saline, so as to eliminate floating cells from the stainless steel board. Thereafter, the board was placed in 400 ml of a sterilized normal saline contained in a vessel, and the saline solution was then stirred with a stirrer (1500-1600 rpm; flow rate: approximately 0.7 m/s) at a room temperature for 30 minutes. After completion of the washing treatment, the stainless steel board was placed in a 100-ml beaker containing 40 ml of a sterilized normal saline, and an ultrasonic treatment was then performed at a medium level of output for 5 minutes. After completion of the treatment, the cell suspension was diluted with a sterilized normal saline, as appropriate, and the number of surviving cells was then measured. As a result, the following was clarified. That is, in the case of the single culture of yeast, the number of cells remaining on the stainless steel board was decreased to approximately 25% by the washing treatment. On the other hand, in the case of a stainless steel board on which a biofilm had been formed by the co-culture of yeast with lactic acid bacteria, the decrease percentage in the number of surviving cells remained at 3% or less even after the washing treatment. Yeast existing in the biofilm was hardly released even by the washing treatment, and thus, cells that formed such biofilm were stably retained on the surface of a solid (Figure 9).

### [Example 8] Observation of structure of biofilm formed by co-culture under scanning electron microscope

Lactic acid bacteria ML11-11 and yeast Y11-43 were inoculated to 20 ml of a YPD medium, and were then subjected to static culture at 30°C, so that a biofilm was allowed to form on the surface of a cover glass established in the culture solution. After the culture for 24 hours, the cover glass was lightly washed, and the structure of the biofilm formed on the surface of the glass was observed under a scanning electron microscope, in a state in which the biofilm remained attached to the glass. As a pre-culture, the lactic acid bacteria were subcultured for two generations at 27°C in an MRS medium (DIFCO). As such pre-culture, the yeast was subcultured for two generations at 27°C in a YPD medium (DIFCO). For the formation of a biofilm, such YPD medium (DIFCO) was always used. In order to prepare biofilm cells used in structural analysis, 20 mL of a YPD medium and MICRO COVER GLASS 22 x 22 (MATSUNAMI) or MICRO SLID GLASS 76 x 26 (MATSUNAMI), which had been sterilized with alcohol, were placed in a sterilized Petri dish. Then, 200 µL of a subculture solution was inoculated thereto in the case of a single culture, and 100 µL each of a subculture solution was inoculated thereto in the case of a co-culture. Thereafter, static culture was carried out at 30°C for 24 hours. After completion of the culture, the medium was removed, and biofilm cells formed on a cover glass were then placed in a 50-ml plastic container, together with the cover glass. Thereafter, the cells were washed with a 0.1 M phosphate buffer twice. Cells adhering to the glass surface even after the washing treatment were defined as biofilm cells. According to ordinary methods, the thus prepared biofilm cells were immobilized with a 2% glutaraldehyde solution and also with a 2% osmic acid solution. Thereafter, the resultant cells were dried using a critical point drier HCP-2 (Hitachi Science Systems, Ltd.). Subsequently, ion coating was performed on the cells using Ion Sputter E-1010 (Hitachi Science Systems, Ltd.), and the resultant was then immobilized on an aluminum specimen support. Using the thus prepared sample, the fine structure of the sample was observed under a scanning electron microscope (3D Real Surface View Microscope VE-8800; KEYENCE Corp.).

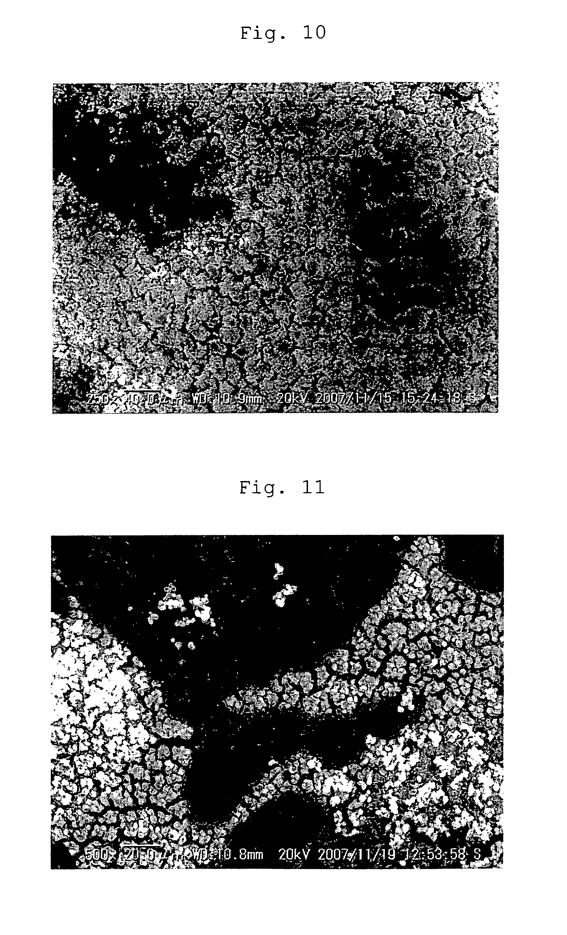

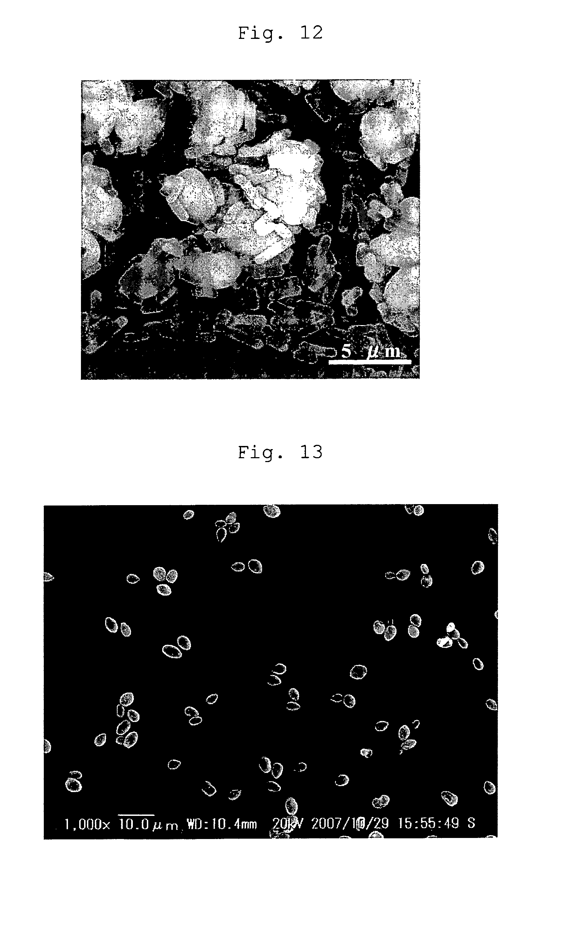

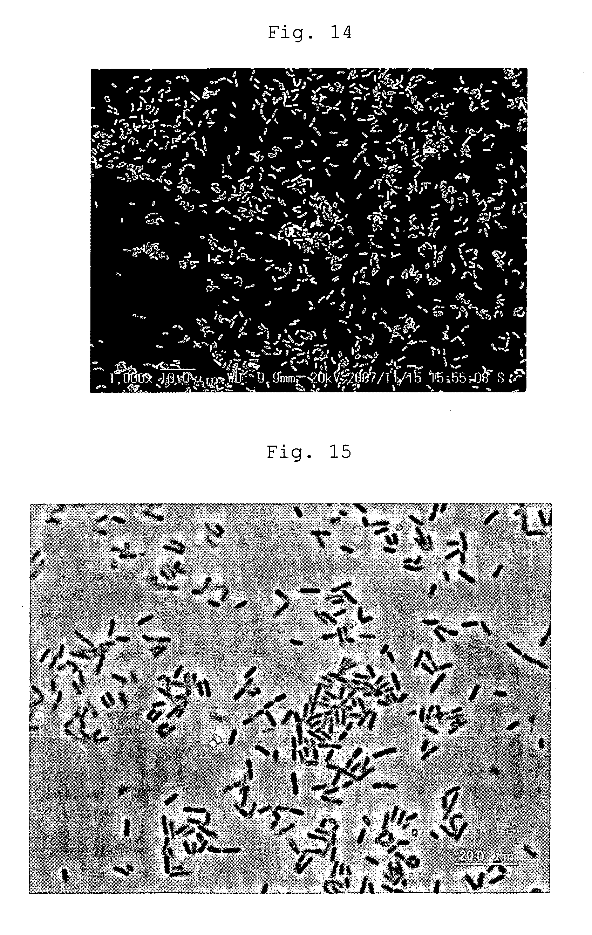

As a result, in the co-culture system, there were found no portions in which yeast cells formed a biofilm by themselves. In contrast, lactic acid bacterial cells tended to adhere to the surface of a solid even on their own (Figure 11). In addition, in a biofilm formed by co-culture, yeast cells were intertwined with lactic acid bacterial cells (Figure 12). On the other hand, in the case of the single culture of yeast, yeast cells hardly adhere to the glass surface (Figure 13). In the case of lactic acid bacteria, however, certain quantities of lactic acid bacterial cells tended to adhere to the glass surface even though the cells were singly cultured, and they tended to form a biofilm (Figure 14).

### [Example 9] Observation of process of forming biofilm by co-culture under phase contrast microscope

Figures 15 to 18 show the results obtained by observing a change over time of biofilm formation by co-culture. Lactic acid bacteria ML11-11 and yeast S. cerevisiae BY4741 were co-cultured in a YPD medium at 30°C in the same manner as described above, so that a biofilm was allowed to form on the surface of a cover glass established in the culture solution. After the initiation of the culture, the cover glass was removed from the medium every 4 hours, and it was then lightly washed. Thereafter, a biofilm formed on the surface of the glass was subjected to phase difference observation using System Microscope BX60 (Olympus Optical Co., Ltd.).

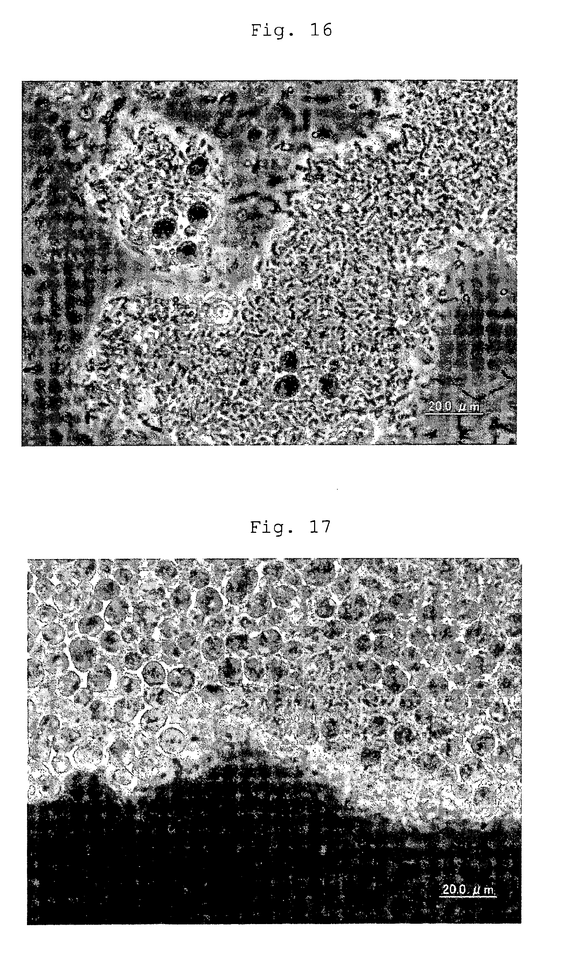

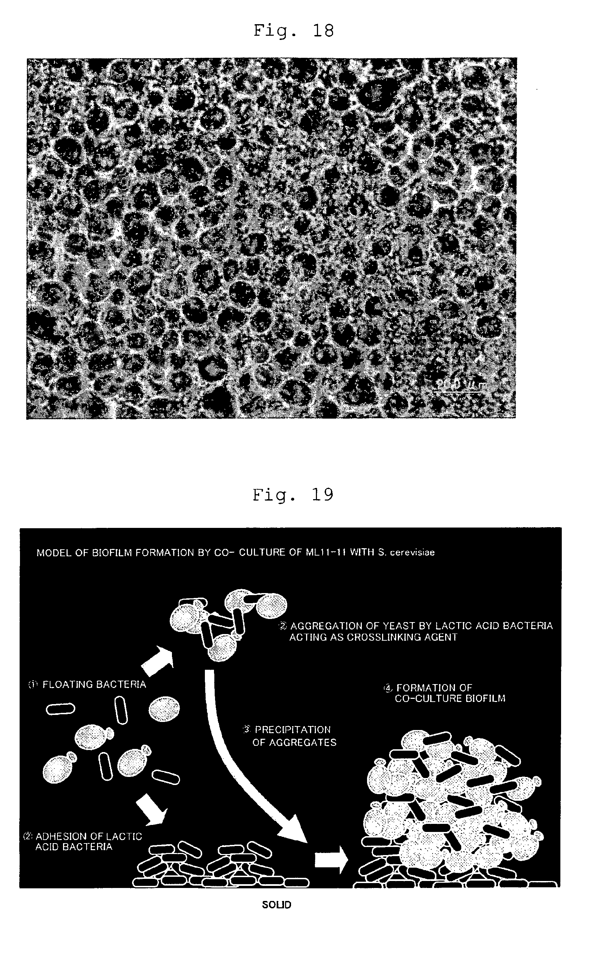

As a result, four hours after the initiation of the culture, lactic acid bacteria began to adhere to the surface of the glass, but yeast hardly adhered thereto (Figure 15). Eight hours after the initiation of the culture, lactic acid bacterial cells formed a film-like aggregate on the glass surface. On the other hand, the number of yeast cells constituting a film was extremely small in comparison with lactic acid bacteria. The yeast cells did not adhere to the glass surface, but all of the cells adhered to the upper portion of the lactic acid bacterial aggregate (Figure 16). Subsequently, twelve hours after the initiation of the culture, a rapidly grown biofilm was observed on the glass surface, and a large number of yeast cells began to adhere to the glass surface, using the biofilm formed by lactic acid bacterial cells as a support. However, there were observed no yeast cells that directly adhered to the glass surface (Figure 17). Thereafter, sixteen hours after the initiation of the culture, a high-cell-density biofilm, in which yeast cells were intertwined with lactic acid bacterial cells, was formed on the glass surface (Figure 18).

As shown in Figure 19, these findings strongly suggested that lactic acid bacterial cells first adhere to the surface of a solid to form a thin biofilm, that lactic acid bacteria crosslink yeast cells to form an aggregate of the yeast cells and the lactic acid bacterial cells, and that the aggregate is sedimented and then forms a significant complex biofilm, using, as a support, the lactic acid bacteria biofilm that has previously been formed on the solid surface.

From the aforementioned results, it became clear that the direct contact of yeast cells with lactic acid bacterial cells is essential for the formation of the biofilm of the present invention, and that the biofilm of the present invention "comprises yeast and lactic acid bacteria, **characterized in that** the lactic acid bacterial cells adhere to a solid surface and yeast cells are immobilized on the solid surface via the lactic acid bacterial cells." There have been no reports regarding the formation of a biofilm by crosslink formation caused by the direct contact between the two types of cells. Moreover, there has not been observed a biofilm comprising yeast and lactic acid bacteria, **characterized in that** the lactic acid bacterial cells adhere to the surface of a solid and the yeast cells are immobilized on the solid surface via the lactic acid bacterial cells. Accordingly, the biofilm of the present invention is considered to be a completely novel structure.

This specification includes the contents as disclosed in the specification and/or drawings of Japanese Patent Application (Patent Application No. 2007-198792 and Patent Application No. 2008-047195), which is a priority document of the present application. In addition, all publications, patents and patent applications cited herein are incorporated herein by reference in their entirety.

## Claims

1. A method for producing a biofilm, **characterized in that** it comprises co-culturing lactic acid bacteria belonging to Lactobacillus plantarum with yeast.

2. The method for producing a biofilm according to claim 1, **characterized in that** the lactic acid bacteria belonging to Lactobacillus plantarum are ML11-11 deposited under accession No. NITE BP-376.

3. The method for producing a biofilm according to claim 1, **characterized in that** the lactic acid bacteria belonging to Lactobacillus plantarum have 16S rDNA consisting of a nucleotide sequence showing homology of 97% or more with the nucleotide sequence shown in SEQ ID NO:1.

4. The method for producing a biofilm according to any one of claims 1 to 3, **characterized in that** the yeast belongs to Saccharomyces cerevisiae.

5. The method for producing a biofilm according to any one of claims 1 to 3, **characterized in that** the yeast has 18S rDNA consisting of a nucleotide sequence showing homology of 97% or more with the nucleotide sequence shown in SEQ ID NO:2.

6. A biofilm produced by the method according to any one of claims 1 to 5.

7. A method for producing a useful substance, using lactic acid bacteria that belong to Lactobacillus plantarum and produce such useful substance, or yeast that produces such useful substance, **characterized in that** it comprises co-culturing the lactic acid bacteria with the yeast and collecting a useful substance from the culture.

8. The method for producing a useful substance according to claim 7, **characterized in that** the lactic acid bacteria and the yeast are converted to an immobilized cell mass, using a biofilm formed by the co-culture of both types of microorganisms.

9. The method for producing a useful substance according to claim 7 or 8, **characterized in that** the useful substance is ethanol.

10. The method for producing a useful substance according to any one of claims 7 to 9, **characterized in that** the lactic acid bacteria belonging to Lactobacillus plantarum are ML11-11 deposited under accession No. NITE BP-376.

11. The method for producing a useful substance according to any one of claims 7 to 9, **characterized in that** the lactic acid bacteria belonging to Lactobacillus plantarum have 16S rDNA consisting of a nucleotide sequence showing homology of 97% or more with the nucleotide sequence shown in SEQ ID NO:1.

12. The method for producing a useful substance according to any one of claims 7 to 11, **characterized in that** the yeast belongs to Saccharomyces cerevisiae.

13. The method for producing a useful substance according to any one of claims 7 to 11, **characterized in that** the yeast has 18S rDNA consisting of a nucleotide sequence showing homology of 97% or more with the nucleotide sequence shown in SEQ ID NO:2.

14. A biofilm comprising yeast and lactic acid bacteria, **characterized in that** the lactic acid bacterial cells adhere to a solid surface and yeast cells are immobilized on the solid surface via the lactic acid bacterial cells.

15. The biofilm according to claim 14, **characterized in that** the lactic acid bacteria are ML11-11 deposited under accession No. NITE BP-376.

16. The biofilm according to claim 14, **characterized in that** the lactic acid bacteria have 16S rDNA consisting of a nucleotide sequence showing homology of 97% or more with the nucleotide sequence shown in SEQ ID NO:1.

17. The biofilm according to any one of claims 14 to 16, **characterized in that** the yeast belongs to Saccharomyces cerevisiae.

18. The biofilm according to any one of claims 14 to 16, **characterized in that** the yeast has 18S rDNA consisting of a nucleotide sequence showing homology of 97% or more with the nucleotide sequence shown in SEQ ID NO:2.

19. Lactobacillus plantarum ML11-11 (Accession No. NITE BP-376).


SEQUENCE LISTING

<110> Nihon University
<120> METHOD FOR PRODUCING BIOFILM
<130> FP-112PCT
<150> JP 2007/198792
<151> 2007-7-31
<150> JP 2008/47195
<151> 2008-2-28
<160> 2
<170> PatentIn Ver. 2.1
<210> 1
<211> 538
<212> DNA
<213> Lactobacillus plantarum
<400> 1
gcccagaagc gggggataac acctggaaac agatgctaat accgcataac aacttggacc 60
gcatggtccg agcttgaaag atggcttcgg ctatcacttt tggatggtcc cgcggcgtat 120
tagctagatg gtggggtaac ggctcaccat ggcaatgata cgtagccgac ctgagagggt 180
aatcggccac attgggactg agacacggcc caaactccta cgggaggcag cagtagggaa 240
tcttccacaa tggacgaaag tctgatggag caacgccgcg tgagtgaaga agggtttcgg 300
ctcgtaaaac tctgttgtta aagaagaaca tatctggcag gcggtttttt aagtctgatg 360
tgaaagcctt cggctcaacc gaagaagtgc atcggaaact gggaaacttg agtgcagaag 420
aggacagtgg aactccatgt gtagcggtga aatgcgtaga tatatggaag aacaccagtg 480
gcgaaggcgg ctgtctggtc tgtaactgac gctgaggctc gaaagtatgg gtagcaaa 538
<210> 2
<211> 527
<212> DNA
<213> Saccharomyces cerevisiae
<400> 2
tcagttatcg tttatttgat agttccttta ctacatggta taactgtggt aattctagag 60
ctaatacatg cttaaaatct cgaccctttg gaagagatgt atttattaga taaaaaatca 120
atgtcttcgg actctttgat gattcataat aacttttcga atcgcatggc cttgtgctgg 180
cgatggttca ttcaaatttc tgccctatca actttcgatg gtaggatagt ggcctaccat 240
ggtttcaacg ggtaacgggg aataagggtt cgattccgga gagggagcct gagaaacggc 300
taccacatcc aaggaaggca gcaggcgcgc aaattaccca atcctaattc agggaggtag 360
tgacaataaa taacgataca gggcccattc gggtcttgta attggaatga gtacaatgta 420
aataccttaa cgaggaacaa ttggagggca agtctggtgc cagcagccgc ggtaattcca 480
gctccaatag cgtatattaa agttgttgca gttaaaaagc tcgtagt 527

SEQUENCE LISTING
<110> Nihon University
<120> METHOD FOR PRODUCING BIOFILM
<130> FP-112PCT
<150> JP 2007/198792
<151> 2007-7-31
<150> JP 2008/47195
<151> 2008-2-28
<160> 2
<170> PatentIn Ver. 2.1
<210> 1
<211> 538
<212> DNA
<213> Lactobacillus plantarum
<400> 1
gcccagaagc gggggataac acctggaaac agatgctaat accgcataac aacttggacc 60
gcatggtccg agcttgaaag atggcttcgg ctatcacttt tggatggtcc cgcggcgtat 120
tagctagatg gtggggtaac ggctcaccat ggcaatgata cgtagccgac ctgagagggt 180
aatcggccac attgggactg agacacggcc caaactccta cgggaggcag cagtagggaa 240
tcttccacaa tggacgaaag tctgatggag caacgccgcg tgagtgaaga agggtttcgg 300
ctcgtaaaac tctgttgtta aagaagaaca tatctggcag gcggtttttt aagtctgatg 360
tgaaagcctt cggctcaacc gaagaagtgc atcggaaact gggaaacttg agtgcagaag 420
aggacagtgg aactccatgt gtagcggtga aatgcgtaga tatatggaag aacaccagtg 480
gcgaaggcgg ctgtctggtc tgtaactgac gctgaggctc gaaagtatgg gtagcaaa 538
<210> 2
<211> 527
<212> DNA
<213> Saccharomyces cerevisiae
<400> 2
tcagttatcg tttatttgat agttccttta ctacatggta taactgtggt aattctagag 60
ctaatacatg cttaaaatct cgaccctttg gaagagatgt atttattaga taaaaaatca 120
atgtcttcgg actctttgat gattcataat aacttttcga atcgcatggc cttgtgctgg 180
cgatggttca ttcaaatttc tgccctatca actttcgatg gtaggatagt ggcctaccat 240
ggtttcaacg ggtaacgggg aataagggtt cgattccgga gagggagcct gagaaacggc 300
taccacatcc aaggaaggca gcaggcgcgc aaattaccca atcctaattc agggaggtag 360
tgacaataaa taacgataca gggcccattc gggtcttgta attggaatga gtacaatgta 420
aataccttaa cgaggaacaa ttggagggca agtctggtgc cagcagccgc ggtaattcca 480
gctccaatag cgtatattaa agttgttgca gttaaaaagc tcgtagt 527